# EUROPEAN PATENT APPLICATION

(11) **EP 2 754 453 A1**
(43) Date of publication of application: **16.07.2014**
(21) Application number: 13150937.4
(22) Date of filing: 11.01.2013
(51) Int. Cl.: A61K 47/48, A61P 35/00, C07K 7/08, A61K 38/10

(54) **Conjugate of a cationic antimicrobial peptide and of a fatty acid for use as an anti-cancer agent**

(71) Applicant: UNIVERSITY COLLEGE DUBLIN, Dublin 4 (IE); Royal College of Surgeons in Ireland, Dublin 2 (IE)
(72) Inventor: O'Connor, Kevin, Dublin, D4 (IE); Gallagher, William, Dublin, D4 (IE); Byrne, Annette, Dublin, D2 (IE); Devocelle, Marc, Dublin, D2 (IE); Szwej, Emilia, Dublin, D4 (IE); O'Connor, Stephen, Dublin, D4 (IE)
(74) Representative: Purdylucey Intellectual Property

(57) **Abstract**

A conjugate of a D-peptide and a (R)-3-substituted fatty acid is described. The D-peptide has a sequence of SEQUENCE ID NO: 1 or is a cytotoxic variant thereof having at least 80% sequence identity with SEQUENCE ID NO: 1. The conjugate exhibits anti-proliferative activity that is greater than the peptide, exhibits improved cellular update, and exhibits selectivity for cancer cells over normal cells.

SEQUENCE LISTING

<110>  University College Dublin
       Royal College of Surgeons in Ireland

<120>  A peptide conjugate suitable for use as an anti-cancer agent.

<130>  P11090EP00

<160>  3

<170>  PatentIn version 3.5

<210>  1
<211>  18
<212>  PRT
<213>  Artificial Sequence

<220>
<223>  cytotoxic peptide DP18L

<400>  1

Lys Trp Lys Leu Phe Lys Lys Leu Pro Lys Phe Leu His Leu Ala Lys
1               5                   10                  15

Lys Phe

<210>  2
<211>  18
<212>  PRT
<213>  Artificial Sequence

<220>
<223>  cytoxic peptide P18

<400>  2

Lys Trp Lys Leu Phe Lys Lys Ile Pro Lys Phe Leu His Leu Ala Lys
1               5                   10                  15

Lys Phe

<210>  3
<211>  17
<212>  PRT
<213>  Artificial Sequence

<220>
<223>  cytoxic peptide P17

<400>  3

Lys Trp Lys Phe Lys Lys Leu Pro Lys Phe Leu His Leu Ala Lys Lys
1               5                   10                  15

Phe

## Description

### Technical Field

The invention relates to a peptide conjugate suitable for use as an anti-cancer agent. The invention also relates to a peptide conjugate for use in a method of treating or preventing cancer.

### Background to the Invention

Antimicrobial peptides, also components of biomaterials, are found in many species (e.g. insects, fish, amphibians and mammals) and play an important role in host innate immunity to microbial pathogens. While initially studied for their antibacterial properties, peptides have become the focus of increased research activity as anti-cancer agents. The cytotoxic effect of cationic antimicrobial peptides (CAPs) on microorganisms and neoplastic cells is largely believed to be a function of their amphipathic nature and secondary structure. CAPs are a class of natural macromolecules and their synthetic analogues that exhibit selective cytotoxicity against a broad spectrum of human cancer cells, including neoplastic cells that have acquired a multidrug resistant phenotype. P18 (KWKLFKKIPKFLHLAKKF) is a hybrid L-peptide of the amino-terminal α-helical region of the 37 amino acid silk moth peptide, Cecropin A, and the amino-terminal α-helical hydrophobic region of the 23 amino acid African claw frog peptide Magainin 2. P18 displays cytotoxic activity against human cancer cell lines (e.g. MDA-MB-231 and Jurkat), but does not exhibit any significant hemolysis.

It is an object of the invention to provide an improved anti-cancer peptide moiety.

### Statements of Invention

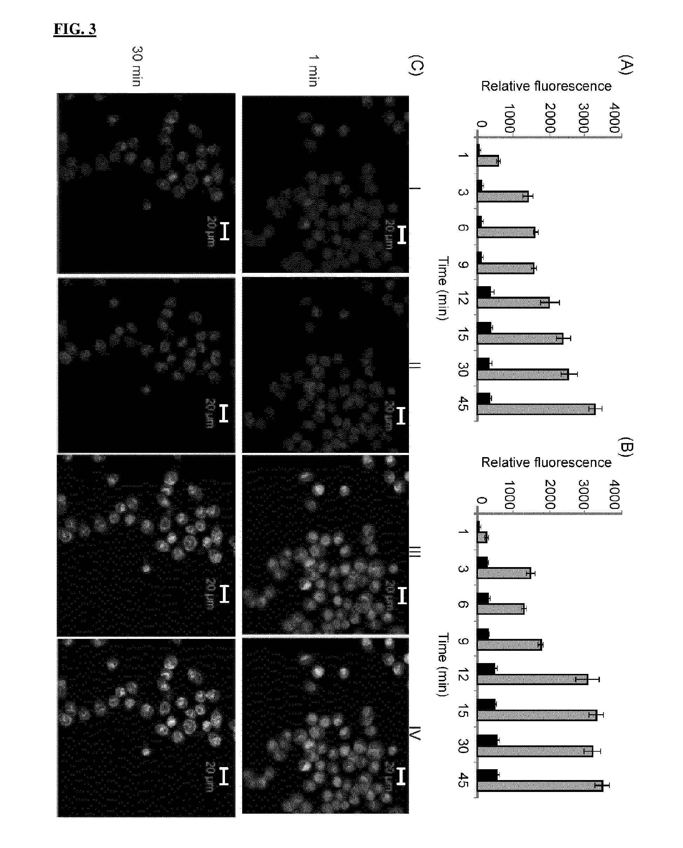

The invention is based on a conjugate of a peptide comprising a cytotoxic peptide DP18L (SEQUENCE ID NO: 1) or cytotoxic variants thereof, and a substituted fatty acid, especially a (R)-3-substitutedfatty acid, and ideally a (R)-3-hydroxylated fatty acid. The conjugate molecules of the invention display improved cytotoxicity compared to the peptide on its own (IC50 of 2.5-5µM compared to 10-20µM), and improved uptake into cells (Figs 3A and 3B). In particular, the (R)-3-hydroxylated fatty acid -peptide conjugates shows selectivity for proliferating cells compared to non-proliferating cells.

Accordingly, the invention provides a conjugate of a peptide and a substituted fatty acid, in which the peptide has a sequence of SEQUENCE ID NO: 1 or a cytotoxic variant thereof having at least 80% sequence identity with SEQUENCE ID NO: 1.

Typically, the substituted fatty acid is an (R)-3-substituted fatty acid or an ω-substituted fatty acid. Ideally, the substituted fatty acid is a (R)-3-hydroxylated fatty acid. The latter conjugates have been shown to show selectivity for cancer cells over normal cells.

Suitably, the fatty acid is substituted with a group selected from hydroxyl, nitro, halogen or mesyalte. Conjugates comprising (R)-3-substituted fatty acids, in which the substituent is selected from chloro, nitro, or mesylate demonstrate the same anti-proliferative activity against MiaPaCa and HeLa cells as the R10DP18L conjugate described below (conjugate comprising hydroxydecanoic acid).

The invention also provides a pharmaceutical composition comprising a conjugate of the invention and a pharmaceutically acceptable carrier.

The invention also relates to a conjugate of the invention for use as a medicament or in a method of treating or preventing cancer in a mammal.

The invention also relates to a peptide, ideally a D-peptide, comprising the sequence DP18L (SEQUENCE ID NO: 1), or a cytoxic variant thereof having at least 80% sequence identity with SEQUENCE ID NO: 1.

### Brief Description of the Figures

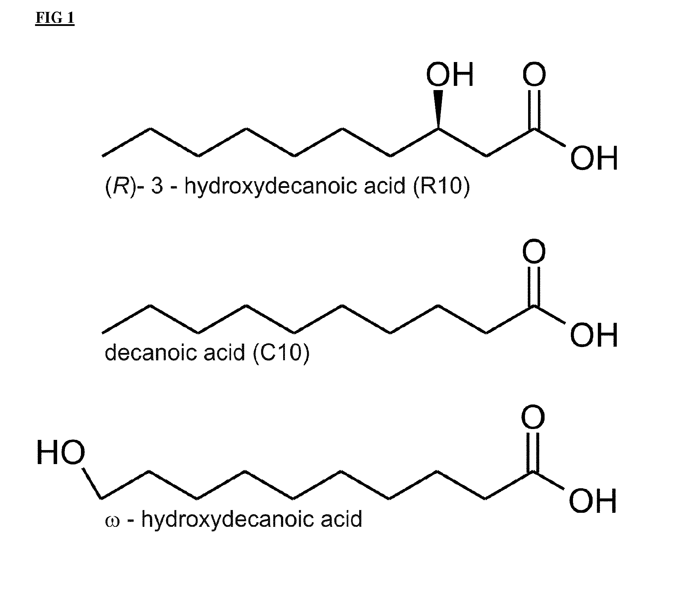

**FIG. 1****.** Chemical structure of (*R*)-3-hydroxydecanoic acid, decanoic acid, and ω-hydroxydecanoic acid which were individually conjugated to peptides in the current study.

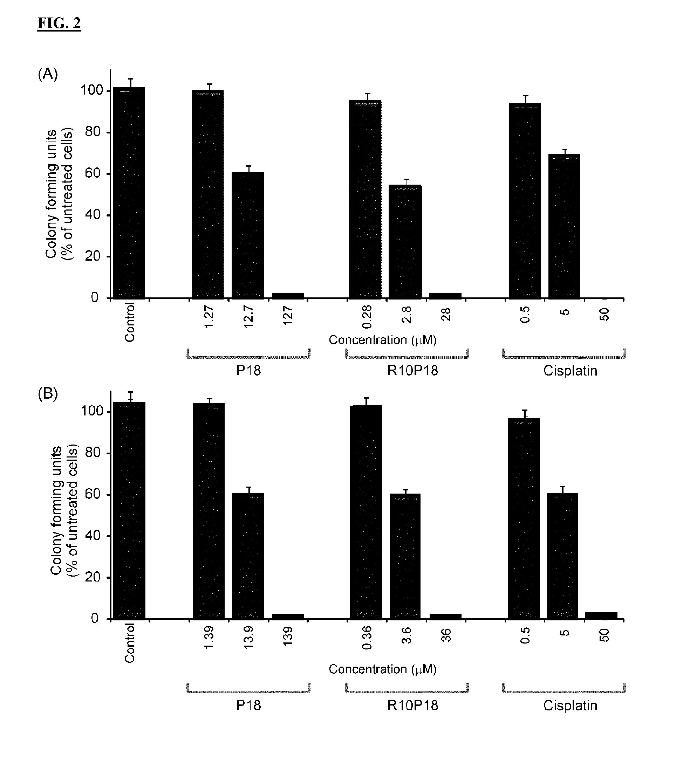

**FIG. 2****.** The effect of peptides DP18L and (*R*)-3-hydroxydecanoic acid derivative R10DP18L on the proliferation of A) HeLa cells and B) MiaPaCa cells when treated at the IC₅₀ value for each test compound and at concentration 10 fold above and below the IC₅₀ value. Data shown is the average of at least three experiments performed in triplicate. Control refers to untreated cells. Cisplatin was a treatment control.
**FIG. 3****.** Uptake and localization of biotinylated peptides in human cells. Cells were treated at approximately the IC50 value for each test compound i.e. A) HeLa cells were treated with 3.3 µM of DP18L (■) and 2.4 µM R10DP18L ( 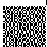 ) B) MiaPaCa cells were treated with 3.5 µM of DP18L (■) and 3.6 µM of R10DP18L ( 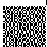 ). C) Biotin-DP18L co-localisation with mitochondria in HeLa cells. HeLa cells were cultured in the presence of 11.5 µM biotinylated-P18L for 1 min (Top row) and for 30 min (bottom row) Column I shows the biotinylated P18L stained with Streptavidin Texas Red®; Column II shows the nucleus stained with DAPI; Column III shows the mitochondria stained with MitoTracker® Green and Column IV shows regions of yellow observed by overlay of the three individual images. Data for A and B is the average of three independent experiments performed in triplicate. Images for panel C are representative of three independent experiments performed in triplicate.

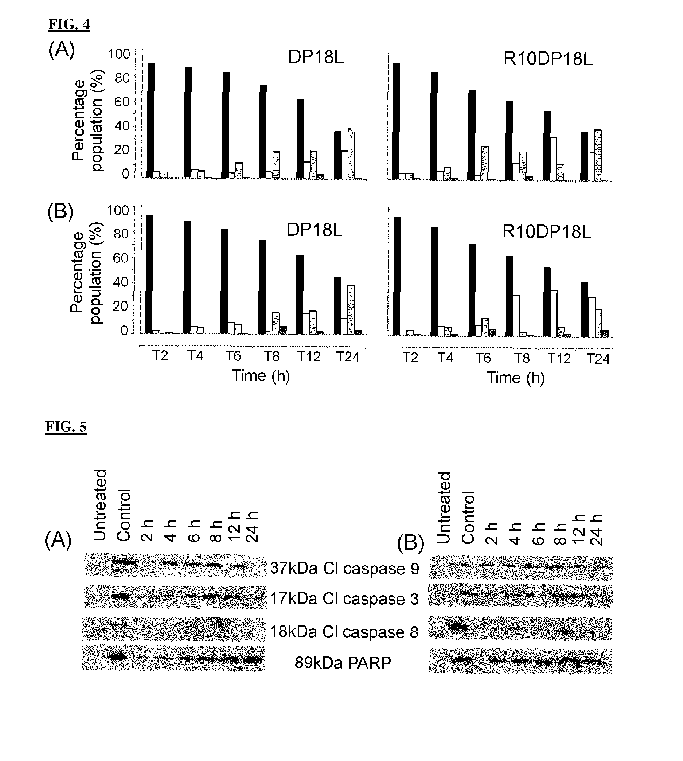

**FIG. 4****.** Induction of an apoptotic mode of cell death following peptide (DP18L and R10DP18L) treatment in A) HeLa cells and B) MiaPaCa cells. The percentage of cell populations that are viable (■), in early apoptosis (D), late apoptosis ( 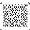 ), and necrosis ( 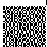 ). Data is the average of three experiments performed in triplicate.
**FIG. 5****.** DP18L induces apoptotic cell death in HeLa (A) and MiaPaCa (B) cells which involves the activation of caspase 3, 9 and the caspase substrate PARP. Caspase 8 was not detected at significant levels in either cell line indicating the intrinsic pathway of apoptosis. Untreated cells were used and positive controls were Jurkat cells treated with 25 µM etoposide for 5 h. Images were similar for all the different compounds tested.

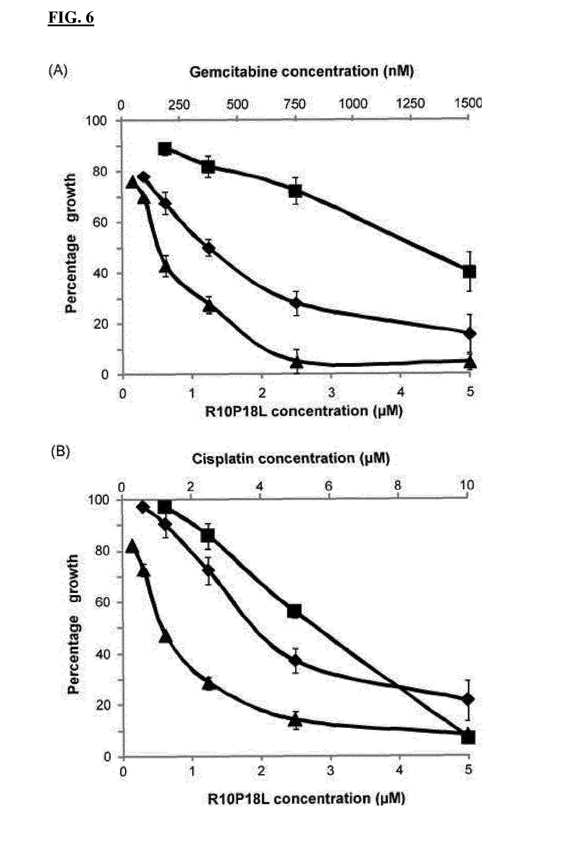

**FIG. 6****.** Examples of synergy between R10DP18L and chemotherapeutic agents. A) Synergy (CI=0.7): WM793 (melanoma) cells incubated with R10DP18L (■), gemcitabine (□), and gemcitabine in combination with R10DP18L (▲). B) Strong synergy (CI=0.3): A549 (lung) cancer cells incubated with R10DP18L (■), cisplatin (□), and cisplatin in combination with R10DP18L (▲). Data is the average of three independent experiments performed in triplicate.

### Detailed Description of the Invention

The conjugate of the invention comprises the cytotoxic peptide DP18L having an amino acid sequence:
KWKLFKKLPKFLHLAKKF (SEQUENCE ID NO: 1).

The conjugate may also comprise cytotoxic variants of SEQUENCE ID NO: 1 having one, two or three amino acid changes by substitution, addition or deletion. Typically, the cytotoxic variant has at least 80%, and ideally at least 90% or 95%, sequence identity with SEQUENCE ID NO: 1. The term "cytotoxic" as applied to the variant peptides should be understood to mean a variant peptide having an IC₅₀ value of less than 10µM (more preferably less than 9µM, 8µM, 7µM, 6µM, or 5µM) against HeLa cells in the anti-proliferative assay described below. An example of a cytotoxic variant is the known cytoxic peptide P18:
KWKLFKKIPKFLHLAKKF (SEQUENCE ID NO: 2).

A further cytotoxic variant is the peptide P17 L having the sequence:
KWKFKKLPKFLHLAKKF (SEQUENCE ID NO: 3)

A conjugate of this peptide, R10P17L (peptide conjugated to (R)-3-hydroxy decanoic acid), has similar antiproliferative activity to the conjugate R10P18L, and even greater selectivity for cancer cells.

The "percent sequence identity" of two amino acid sequences is determined using the algorithm of Karlin and Altschul Proc. Natl. Acad. Sci. USA 87:2264-68, 1990, modified as in Karlin and Altschul Proc. Natl. Acad. Sci. USA 90:5873-77, 1993. Such an algorithm is incorporated into the NBLAST and XBLAST programs (version 2.0) of Altschul, et al. J. Mol. Biol. 215:403-10, 1990. BLAST protein searches can be performed with the XBLAST program, score=50, wordlength=3 to obtain amino acid sequences homologous to the protein molecules of the invention. Where gaps exist between two sequences, Gapped BLAST can be utilized as described in Altschul et al., Nucleic Acids Res. 25(17):3389-3402, 1997. When utilizing BLAST and Gapped BLAST programs, the default parameters of the respective programs (e.g., XBLAST and NBLAST) can be used.

The conjugate described above can be in the free form or in the form of salt, if applicable. A salt, for example, can be formed between an anion and a positively charged group (e.g., amino) on a protein-polymer conjugate of this invention. Suitable anions include chloride, bromide, iodide, sulfate, nitrate, phosphate, citrate, methanesulfonate, trifluoroacetate, and acetate. Likewise, a salt can also be formed between a cation and a negatively charged group (e.g., carboxylate) on a conjugate of this invention. Suitable cations include sodium ion, potassium ion, magnesium ion, calcium ion, and an ammonium cation such as tetramethylammonium ion.

In addition, the conjugate may have one or more double bonds, or one or more asymmetric centers. Such a conjugate can occur as racemates, racemic mixtures, single enantiomers, individual diastereomers, diastereomeric mixtures, and cis- or trans- or E- or Z- double bond isomeric forms.

The peptide component of the conjugate of the invention is preferably a D-peptide.

The term "substituted fatty acid" as used herein refers to a C₄ to C₂₀ fatty acid that is substituted with a moiety, preferably selected from hydroxyl, nitro, halogen, or mesylate. Preferably, the substitution is selected from a (R)-3 substitution and an ω-substitution. More preferably, the substitution is selected from a (R)-3 substitution. Ideally, the substitution is selected from a (R)-3 hydroxy substitution. Preferably the substituted C₄ to C₂₀ fatty acid is a substituted C₆-C₂₀, C₆-C₁₈, C₆-C₁₆, C₆-C₁₄, C₆-C₁₂, or C₈-C₁₂ fatty acid. Suitably, the substituted fatty acid is saturated, although conjugates comprising unsaturated fatty acids (in cis or trans form) may form part of the invention. The substituted fatty acid may preferably be a decanoic acid moiety, preferably a hydroxylated decanoic acid. In one embodiment, the C₈ to C₁₂ fatty acid is selected from decanoic acid, (R)-3-hydroxydecanoic acid, and ω-hydroxydecanoic acid. The fatty acid part of the conjugate of the invention may also be unsaturated, for example mono-unsaturated, di-unsaturated, or tri-unsaturated.

The term "peptide" used herein refers to a polymer composed of up to 50 amino acid monomers via peptide bond linkage. These peptides can be prepared by conventional methods, i.e., chemical synthesis or recombinant technology. When necessary, any of the cytotoxic peptides employed in the conjugate of the invention can be chemically modified to increase their stability. A chemically modified peptide or a peptide analog includes any functional chemical equivalent of the peptide characterized by its increased stability and/or efficacy in vivo or in vitro in respect of the practice of the invention. The term peptide analog also refers to any amino acid derivative of a peptide as described herein. A peptide analog can be produced by procedures that include, but are not limited to, modifications to side chains, incorporation of unnatural amino acids and/or their derivatives during peptide synthesis and the use of cross-linkers and other methods that impose conformational constraint on the peptides or their analogs. Examples of side chain modifications include modification of amino groups, such as by reductive alkylation by reaction with an aldehyde followed by reduction with NaBH₄; amidation with methylacetimidate; acetylation with acetic anhydride; carbamylation of amino groups with cyanate; trinitrobenzylation of amino groups with 2, 4, 6, trinitrobenzene sulfonic acid (TNBS); alkylation of amino groups with succinic anhydride and tetrahydrophthalic anhydride; and pyridoxylation of lysine with pyridoxa-5'-phosphate followed by reduction with NABH₄. The guanidino group of arginine residues may be modified by the formation of heterocyclic condensation products with reagents such as 2,3-butanedione, phenylglyoxal and glyoxal. The carboxyl group may be modified by carbodiimide activation via o-acylisourea formation followed by subsequent derivatization, for example, to a corresponding amide. Sulfhydryl groups may be modified by methods, such as carboxymethylation with iodoacetic acid or iodoacetamide; performic acid oxidation to cysteic acid; formation of mixed disulphides with other thiol compounds; reaction with maleimide; maleic anhydride or other substituted maleimide; formation of mercurial derivatives using 4-chloromercuribenzoate, 4-chloromercuriphenylsulfonic acid, phenylmercury chloride, 2-chloromercuric-4-nitrophenol and other mercurials; carbamylation with cyanate at alkaline pH. Tryptophan residues may be modified by, for example, oxidation with N-bromosuccinimide or alkylation of the indole ring with 2-hydroxy-5-nitrobenzyl bromide or sulphonyl halides. Tryosine residues may be altered by nitration with tetranitromethane to form a 3-nitrotyrosine derivative. Modification of the imidazole ring of a histidine residue may be accomplished by alkylation with iodoacetic acid derivatives or N-carbethoxylation with diethylpyrocarbonate. Examples of incorporating unnatural amino acids and derivatives during peptide synthesis include, but are not limited to, use of norleucine, 4-amino butyric acid, 4-amino-3-hydroxy-5-phenylpentanoic acid, 6-aminohexanoic acid, t-butylglycine, norvaline, phenylglycine, ornithine, sarcosine, 4-amino-3-hydroxy-6-methylheptanoic acid, 2-thienyl alanine and/or D-isomers of amino acids.

As used in this disclosure, "conjugated" means two entities (here a cytoxic peptide as described above and a substituted fatty acid moiety) are associated with sufficient affinity that the therapeutic benefit of the association between the two entities is realized. Conjugation can be achieved by covalent or noncovalent bonding, as well as by other forms of association, such as entrapment of one entity on or within the other, or of either or both entities on or within a third entity (e.g., a micelle). Preferably the peptide and fatty acid are conjugated via an amide bond between a carboxyl of the fatty acid and an N-terminal amino of the peptide.

The conjugate of the invention preferably has a very high purity. Namely, in 80% or more of the conjugate molecules, the fatty acid moiety is linked to the same nitrogen atom of the N-terminus of the same peptide moiety. In other words, in at least 80% of the conjugate molecules, the peptide moiety is identical in all aspects, including its sequence and its bonding position to the fatty acid moiety.

Thus, one aspect of this invention relates to a method of administering an effective amount of one or more of the above-described conjugates for treating a disease (e.g. cancer). Specifically, a disease can be treated by administering to a subject one or more of the conjugates in an effective amount. Such a subject can be identified by a health care professional based on results from any suitable diagnostic method.

As used herein, the term "treating" or "treatment" is defined as the application or administration of a composition including a conjugate of the invention to a subject (human or animal), who has a disorder, a symptom of the disorder, a disease or disorder secondary to the disorder, or a predisposition toward the disorder, with the purpose to cure, alleviate, relieve, remedy, or ameliorate the disorder, the symptom of the disorder, the disease or disorder secondary to the disorder, or the predisposition toward the disorder. Typically, the disorder is cancer. "An effective amount" refers to an amount of a conjugate which confers a therapeutic effect on the treated subject. The therapeutic effect may be objective (i.e., measurably by some tests or markers) or subjective (i.e., a subject gives an indication of or feels an effect). Also within the scope of this invention is a pharmaceutical composition contains an effective amount of at least one of the conjugates described above and a pharmaceutical acceptable carrier. Further, this invention includes a method of administering an effective amount of one or more of conjugates to a patient with one or more diseases. Effective doses will vary, as recognized by those skilled in the art, depending on, e.g., the rate of hydrolysis of a protein-polymer conjugate, the types of diseases to be treated, the route of administration, the excipient usage, and the possibility of co-usage with other therapeutic treatment.

To practice the method of the present invention, a composition having one or more of the above-mentioned compounds can be administered parenterally, orally, nasally, rectally, topically, or buccally. The term "parenteral" as used herein refers to subcutaneous, intracutaneous, intravenous, intramuscular, intraarticular, intraarterial, intrasynovial, intrasternal, intrathecal, intralesional, intraperitoneal, intratracheal or intracranial injection, as well as any suitable infusion technique. A sterile injectable composition can be a solution or suspension in a non-toxic parenterally acceptable diluent or solvent, such as a solution in 1,3-butanediol. Among the acceptable vehicles and solvents that can be employed are mannitol, water, Ringer's solution, and isotonic sodium chloride solution. In addition, fixed oils are conventionally employed as a solvent or suspending medium (e.g., synthetic mono-or di-glycerides). Fatty acid, such as oleic acid and its glyceride derivatives are useful in the preparation of injectables, as are natural pharmaceutically acceptable oils, such as olive oil or castor oil, especially in their polyoxyethylated versions. These oil solutions or suspensions can also contain a long chain alcohol diluent or dispersant, or carboxymethyl cellulose or similar dispersing agents. Other commonly used surfactants such as Tweens or Spans or other similar emulsifying agents or bioavailability enhancers which are commonly used in the manufacture of pharmaceutically acceptable solid, liquid, or other dosage forms can also be used for the purpose of formulation.

A composition for oral administration can be any orally acceptable dosage form including capsules, tablets, emulsions, and aqueous suspensions, dispersions, and solutions. In the case of tablets, commonly used carriers include lactose and corn starch. Lubricating agents, such as magnesium stearate, are also typically added. For oral administration in a capsule form, useful diluents include lactose and dried corn starch. When aqueous suspensions or emulsions are administered orally, the active ingredient can be suspended or dissolved in an oily phase combined with emulsifying or suspending agents. If desired, certain sweetening, flavoring, or coloring agents can be added. A nasal aerosol or inhalation composition can be prepared according to techniques well known in the art of pharmaceutical formulation. For example, such a composition can be prepared as a solution in saline, employing benzyl alcohol or other suitable preservatives, absorption promoters to enhance bioavailability, fluorocarbons, and/or other solubilizing or dispersing agents known in the art. A composition having one or more of the above-described compounds can also be administered in the form of suppositories for rectal administration.

A pharmaceutically acceptable carrier is routinely used with one or more active above-mentioned compounds. The carrier in the pharmaceutical composition must be "acceptable" in the sense that it is compatible with the active ingredient of the composition (and preferably, capable of stabilizing the active ingredient) and not deleterious to the subject to be treated. One or more solubilizing agents can be utilized as pharmaceutical excipients for delivery of an above-mentioned compound. Examples of other carriers include colloidal silicon oxide, magnesium stearate, cellulose, sodium lauryl sulfate, and D&C Yellow # 10.

The invention also relates to a pharmaceutical composition comprising the conjugate of the invention, a chemotherapeutic agent, and a pharmaceutically acceptable excipient. The invention also relates to a method for the treatment or prevention of cancer in a mammal comprising administering to the mammal, together or separately, a conjugate of the invention and a chemotherapeutic agent.

In this specification, the term "chemotherapeutic agent" should be taken to mean an agent that induces cancerous cells to commit to cell death. Suitable chemotherapeutic agents will be known to those skilled in the art. Such chemotherapeutic agents include but are not limited to; alkylating agents, anti-metabolites, plant alkyloids and terpenoids, topoisomerase inhibitors, anti-tumour antibiotics,DNA-binding heavy metal ion-based complexes including but not limited to the platinum-based complexes cisplatin, carboplatin and oxaliplatin, and histone deacetylase (HDAC) inhibitors including hydroxamate-type HDAC inhibitors (SAHA, Pabinostat, Belinostat) and benzamide-type HDAC inhibitors (the details of which will be well known to those skilled in the art. Examples of suitable chemotherapeutic anti-metabolites include, purine analogues not limited to azathoprine, mercaptopurine, tioguanine and fludarabine; pyrimidine analogues not limited to 5-fluorouracil (5-FU), floxuridine and cytosine arabinoside; antifolates not limited to methotrexate, trimethoprim, pyrimethamine and pemetrexed. Suitably, the chemotherapeutic agent is a DNA damaging agent (to include DNA-binding agent). Preferably, it is a pyrimidine analogue, examples of which are provided above. Ideally, it is 5-FU.

Typically, the cancer is selected from the group comprising: esophagogastric cancer; fibrosarcoma; myxosarcoma; liposarcoma; chondrosarcoma; osteogenic sarcoma; chordoma; angiosarcoma; endotheliosarcoma; lymphangiosarcoma; lymphangioendotheliosarcoma; synovioma; mesothelioma; Ewing's tumor; leiomyosarcoma; rhabdomyosarcoma; colon carcinoma; pancreatic cancer; breast cancer; ovarian cancer; prostate cancer; squamous cell carcinoma; basal cell carcinoma; adenocarcinoma; sweat gland carcinoma; sebaceous gland carcinoma; papillary carcinoma; papillary adenocarcinomas; cystadenocarcinoma; medullary carcinoma; bronchogenic carcinoma; renal cell carcinoma; hepatoma; bile duct carcinoma; choriocarcinoma; seminoma; embryonal carcinoma; Wilms' tumor; cervical cancer; uterine cancer; testicular tumor; lung carcinoma; small cell lung carcinoma; bladder carcinoma; epithelial carcinoma; glioma; astrocytoma; medulloblastoma; craniopharyngioma; ependymoma; pinealoma; hemangioblastoma; acoustic neuroma; oligodendroglioma; meningioma; melanoma; retinoblastoma; and leukemias. Typically, treatment of the cancer entails reducing one or more of survival, proliferation and migration of, or invasion by, cancer cells.

### EXPERIMENTAL

### Materials and methods

### Production of PHA, (R)-3-hydroxyalkanoic acids, peptides and peptide derivatives

PHA was synthesized by supplying glucose to *Pseudomonas putida* CA-3 in a stirred tank reactor for 48 h at 30°C under conditions of nitrogen limitation and carbon excess (ratio C:N 18:1) [1]. Cells were lyophilized in order to remove any water and the PHA polymer was extracted in acetone at room temperature for 18 h-24 h [2]. The PHA was methanolyzed and the resulting methylated monomer samples were analyzed on a Agilent 6890N gas chromatograph fitted with 5973 series inert mass spectrophotometer (Agilent Technologies, Santa Clara, USA) equipped with a BP21 capillary column (25 m by 0.25 mm, 0.32-µm film thickness; SGE Analytical Sciences) with temperature programming (120°C for 5 min; temperature ramp of 3°C per min; 180°C for 10 min) to determine monomer composition [3]. The (*R*)-3-hydroxydecanoic acid monomer was purified from the biopolymer methanolysis mixture using the preparative reversed-phase column chromatography and characterized as described by Ruth and co-workers [4].

Unless otherwise specified, the D-peptide sequences were assembled by solid phase synthesis on a Rink amide-MBHA resin, yelding C-terminally amidated peptides, using Fmoc/*t*-Bu-strategy on a 433A peptide synthesizer (Applied Biosystems, Warrington, UK) as described previously [5]. Biotinylated peptides were synthesized by N-terminal modification of the sequences with Lys(□-biotinyl) and amidation of its *N*^{□}-amino group with (R)-3-hydroxydecanoic acid. Fmoc-Lys(biotin)-OH was added to the polymer-bound, N-terminally deprotected peptides by PyBop/HOBt/DIEA coupling chemistry. The purity of the peptides was above 95%, as assessed by analytical reversed phase high performance liquid chromatography on a Varian HPLC system equipped with Phenomenex C18 Gemini column (4.6 mmD x 250 mmL; Phenomenex, Torrance, USA). Matrix-assisted laser desorption/ionization tandem time-of-flight mass spectrometry on an AB Sciex 4800 Plus MALDI TOF/TOF was used to characterize all peptides with an α-cyano-4-hydroxy-cinnamic acid was used as a matrix.

The conjugation of (*R*)-3-hydroxydecanoic acid (R10), decanoic acid (C10) or ω-hydroxydecanoic acid (ω10) (Fig. 1) to DP18L was through an amide bond between the carboxyl of the fatty acid and the N-terminal amino of a resin-bound peptide as previously described [6]. Briefly, 180 mg of resin primed in dimethylformamide (DMF) was placed in solution of alkanoic acid to be conjugated (0.039 g, 0.4 mmol) in *N*-methyl-2-pyrrolidone (NMP) (5 ml). Reaction mixture also contained 4-(4,6-dimethoxy-1,3,5-triazin-2-yl)-4-methylmorpholinium chloride (DMTMM) (0.08 g, 0.3 mmol) and *N,N*-diisopropylethylamine (DIEA) (75 µl, 0.6 mmol) and was stirred at ambient temperature. After 5 min of reaction the resin was isolated by filtration and washed twice with DMF (2 min, 5 ml) and twice with DCM (2 min, 5 ml). Conjugation was monitored by the qualitative Kaiser test [7].

### Cell culture

The colorectal cancer (HT-29), lung carcinoma (A549) and T-cell leukemia (Jurkat) cell lines were maintained as monolayer cultures in RPMI-1640 supplemented with 100 µg/ml streptomycin, 100 U/ml penicillin and 10% (v/v) foetal bovine serum (FBS) (all from Sigma, Ireland). Human glioma (SNB-19), human pancreatic carcinoma (MiaPaCa) and breast carcinoma (MDA-MB-231) cells were obtained from the American Type Culture Collection (ATCC) and maintained in Dulbecco's Modified Eagle's Medium (DMEM) supplemented with 100 µg/ml streptomycin, 100 U/ml penicillin, 2 mM L-glutamine, and 10% (v/v) FBS. The melanoma cell line (WM793) was originally isolated from a superficial spreading melanoma [8] and maintained in DMEM, supplemented with 10% (v/v) FBS, 50 µg/ml gentamicin and 4 µg/ml insulin (Sigma, Ireland). HeLa cells were obtained from the ATCC and maintained in DMEM supplemented with 100 µg/ml streptomycin, 100 U/ml penicillin, 2 mM L-glutamine, 0.1 mM non-essential amino acids (NEAA, Sigma), and 10% (v/v) FBS. Human umbilical vein endothelial cells (HUVEC) cells were obtained from Cambrex and maintained in an endothelial cell growth medium (Lonza, Dublin Ireland). Human Dermal fibroblast (HDF) cells were obtained from the European Collection of Cell Culture (ECACC) and were maintained in DMEM supplemented with 100 µg/ml streptomycin, 100 U/ml penicillin, 2 mM L-glutamine, and 10% (v/v) FBS. All cells were grown in humidified atmosphere of 95% air and 5% CO₂ at 37°C.

### MTT cell viability assay

Reduction of MTT [(3-(4,5-dimethylthiazol-2-yl)-2,5-diphenyltetrazolium bromide] by cells was assessed colorimetrically as previously described [9]. Assays were carried out after 48 h of cell incubation (72 h for combinational studies) in the media containing test compounds at concentrations ranging from 200 µM to 0.2 µM depending upon compounds being tested.

### Clonogenic assay

Based on MTT assay data HeLa or MiaPaCa cells were treated at a three different concentrations i.e. 10 fold lower than the IC₅₀, at the IC₅₀, and 10 fold higher than the IC₅₀ value. Following treatment, cells were seeded in triplicate in 6-well plates at densities of 500 cells/well. Cells were then allowed to form colonies for approximately 2 weeks. Cells were fixed in 10% neutral-buffered formalin (Sigma, Ireland). Fixing agent was removed, and plates were allowed to air dry, before staining with a 0.25% (w/v) solution of crystal violet

### Apoptosis assay

Apoptosis was determined as per manufacturer's instructions (BD biosciences manual) using an Annexin V staining kit (BD Biosciences Ireland). Apoptosis was characterized by chromatin condensation and fragmentation when examined by fluorescence microscopy. The incidence of apoptosis in each preparation was analyzed by counting 10,000 cells. Treated and control samples were analyzed using Summit software version 4.2 on a CyanADP flow cytometer (DakoCytomation, Cambridge, UK).

### Western blot analysis

Cells were lyzed and protein levels determined using the bicinchoninic acid method (Pierce, Rockford, IL). Protein samples were separated by SDS-PAGE under reducing conditions. Resolved proteins were transferred to methanol-activated polyvinylidenedifluoride (PVDF) membrane (Millipore, Bedford, MA, USA and blocked in 5% (w/v) bovine serum albumin for 1 h at room temperature. Primary antibody was applied to membranes overnight at 4°C (anti-cleaved Caspase 3, 8, 9 and poly ADP-ribose polymerase (PARP), (1:1000 dilution; Cell Signaling Technology, Danvers, USA). Chemiluminescent signal was detected using the Fluorchem FC2 Imaging System (Alpha Innotech, Switzerland). Membranes were stripped and reprobed with anti-β-actin antibodies (1:1000 dilution; Santa-Cruz Biotechnology, Santa Cruz, USA) so as to provide loading control.

### In-vitro uptake studies

### Immunofluorescence and confocal microscopy

To determine the uptake of peptides into cells, HeLa and MiaPaCa cells were incubated in the presence or absence of biotinylated forms of the most active peptides at their respective IC₅₀ values over a 45 min period with periodic sampling over that time. Images were obtained using a LSM510 META confocal laser-scanning microscope (Zeiss, Jena, Germany). MitoTracker Green was excited by a 488 nm argon laser and DAPI was excited by a 364 nm UV laser, while Texas Red was excited by a 543 nm helium neon laser. Ten thousand cells (HeLa and MiaPaCa) were plated out in a 96 well plate and allowed to adhere overnight at 37°C in a 5% CO₂ humidified atmosphere. Cells were treated with MitoTracker green (1:200 dilution (20 µM), 45 min, 37°C) and then exposed to biotinylated-peptides for various time points at the respective IC₅₀ values of the most therapeutically active peptides, at 37°C in a 5% CO₂ humidified atmosphere. Following treatment, cells were washed twice with phosphate buffered saline (PBS), fixed with 3.7% paraformaldehyde for 15 min, washed twice with PBS, and incubated at 4°C overnight in PBS. Cells were then permeabilized with 0.5% Triton X-100 for 5 min followed by an additional two washes with PBS. Plates were then incubated with Streptavidin-Texas Red (1:200 dilution, 1 h, 20°C) (Calbiochem, UK) and protected from light. 4',6-diamidino-2-phenylindole (DAPI) (Sigma, Ireland) (100µl, 1:2000 in PBS, 2 min, 20°C) was added to each well and protected from light for 5 min. Plates were then analyzed using the IN Cell Analyser 1000 for high content analaysis (HCA).

### Automated microscopy and automated data analysis

The IN Cell Analyzer 1000 automated fluorescent imaging system (GE Healthcare, Piscataway, NJ) was used for automated image acquisition. Images were acquired with a 40X objective lens using a 360/20 nm, a 535/20 nm and a 565/20 nm excitation filter, a 460/50nm, a 600/20 nm and a 620/20 nm HQ emission filter, and an exposure time of 25 (DAPI) to 450 (Steptavidin Texas Red) ms (milliseconds) depending upon the wavelength. The instrument acquired images of each well in a 96 well plate with a laser-based autofocus system. To score co-localisation in a high-content throughput fashion, the Multi-Target Analysis algorithm (GE Healthcare, Investigator v3.5) was used to identify individual cells and granules (mitochondria) within these cells. The nucleus was segmented via a collar method (50 µm² minimum areas) with light shading and noise removal to allow 'touching' nuclei to be separated. Granules surrounding the nucleus (mitochondria) were segmented using a multiscale top-hat method measuring granules of 1 to 2 µm in size and using a 'smart masking' method to identify more precisely the boundaries of each segmented granule. The granules within this top hat region were then measured for their fluorescent intensity and the change in the intensity of these granules used to generate a quantifiable measure of co-localisation.

### Combination studies

A549, HeLa, MiaPaCa and WM793 cells were treated individually with cisplatin, gemcitabine, taxotere, and in combination with R10DP18L starting with a maximum concentration of 20 µM, 1.5 µM, 20 nM and 5 µM respectively. The plates were placed in the incubator for 72 h at 37°C 5% CO₂. The IC₅₀ value for the individual and combination studies was determined using CalcuSyn software (Biosoft, Cambridge, UK). Combination indices (CI) were calculated on the basis of the assumption of mutually non-exclusive drug interactions. Synergy is defined as CI < 0.9; additivity as CI > 0.9 but < 1.1; and antagonism as CI > 1.1 [10].

### Statistical analysis

For the statistical analysis, we used a *t*-test to compare between two groups. Differences among the three groups were compared with one-way analysis of variance (ANOVA) and the post hoc Tukey's honest significant difference (Tukey-HSD) test for multiple comparisons. Differences were defined as significant at *p*<0.05. Analysis of variance (ANOVA) was performed to compare one set of double drug combinations with other double drug combinations.

**TABLE 1. Nomenclature of the peptides and derivatives made and tested in this study.**

| Original Peptide | New Peptide Annotation | Modification | Sequence^{a} |
|---|---|---|---|
| P18 | DP18 | D - instead of L- amino acids | Kwklfkkipkflhlakkf (SEQ ID NO: 2) |
| DP18 | DP18L | Substitute Iso with Leu (residue 8) | Kwklfkklpkflhlakkf (SEQ ID NO: 1) |
| DP18L | R10DP18L | Conjugate (*R*)-3-hydroxydecanoic acid to P18L | R10-kwklfkklpkflhlakkf |
| DP18L | C10DP18L | Conjugate decanoic acid to P18L | C10-kwklfkklpkflhlakkf |
| DP18L | ωC10DP18L | Conjugate ω-hydroxydecanoic acid to P18L | ω10-kwklfkklpkflhlakkf |

| | | | |
|---|---|---|---|
| ^{a}Amino acid sequence lower case to indicate D-stereoisomers. | | | |

**TABLE 2. The anti-proliferation activity the synthetic peptide DP18 and its derivatives with cancer cell lines. Values show the micromolar (µM) concentration at which 50% cell growth inhibition occurs (IC₅₀).**

| | HeLa | A549 | Jurkat | MDA | HT-29 | WM793 | SNB-19 | MiaPaCa | HUVEC | HDF |
|---|---|---|---|---|---|---|---|---|---|---|
| DP18 | 18±0.4^{a} | 12.9±1.4 | 8.1±0.1 | 18.6±0.8 | 17.5±0.5 | 22.8±0.8 | 18.6±1.5 | 18.9±1.5 | 100±9 | 30.4±1 |
| R10DP18 | 5.6±0.4 | 7.2±1.2 | 8.8±1.1 | 3.4±0.9 | 6.3±0.8 | 8.5±1.7 | 11.3±0.9 | 7.7±0.2 | 18±1.7 | 7.9±0.8 |
| | | | | | | | | | | |
| DP18L | 12.7±1.2 | 6.2± 0.5 | 4.2±0.6 | 13.4±2.4 | 18.3±0.9 | 18.7±3 | 27.3±1.8 | 13.9±0.6 | 140±18 | 13.8±1.2 |
| R10DP18L | 2.8±0.3 | 2.5±0.7 | 3±0.5 | 3.1±0.8 | 6.6±0.3 | 5.2±1.4 | 6.8±1.4 | 3.6±0.9 | 9.2±0.8 | 7.9±0.4 |
| C10DP18L | 3±0.1 | 3.4±0.2 | 3.8±0.4 | 4.8±0.7 | 3.6±0.5 | 7±0.1 | 4.3±0.9 | 5±1.5 | 3.9±0.4 | 3.2±0.6 |

| | | | | | | | | | | |
|---|---|---|---|---|---|---|---|---|---|---|
| ^{a} Data is an average of three independent determinations performed in triplicate | | | | | | | | | | |

**TABLE 3. Combination Index (CI) for fixed ratio combinations of cisplatin, gemcitabine and taxotere with R10DP18L in A549, HeLa, MiaPaCa and WM793 cells respectively.**

| Combination | A549 | HeLa | MiaPaCa | WM793 |
|---|---|---|---|---|
| Taxotere + R10DP18L | 0.4±0.2^{a} | 0.6±0.1 | 0.7±0.1 | 0.5±0.1 |
| Cisplatin + R10P18L | 0.3±0.1 | 1.2±0.3 | 0.9±0.1 | 0.7±0.1 |
| Gemcitabine + R10P18L | 1.0±0.2 | 1.1±0.2 | 0.5±0.2 | 0.7±0.2 |

| | | | | |
|---|---|---|---|---|
| ^{a} Data is an average of three independent determinations performed in triplicate. | | | | |

The invention is not limited to the embodiments hereinbefore described which may be varied in construction and detail without departing from the spirit of the invention.

### REFERENCES

[1] Ward PG, de Roo G, O'Connor KE. Accumulation of polyhydroxyalkanoate from styrene and phenylacetic acid by Pseudomonas putida CA-3. Appl Environ Microbiol 2005; 71: 2046-52.
[2] Elbahloul Y, Steinbüchel A. Large-scale production of poly(3-hydroxyoctanoic acid) by Pseudomonas putida GPo1 and a simplified downstream process. Appl Environ Microbiol 2009; 75: 643-51.
[3] Lee EY, Choi CY. Gas chromatography-mass spectrometric analysis and its application to a screening procedure for novel bacterial polyhydroxyalkanoic acids containing long chain saturated and unsaturated monomers. J Ferment Bioeng 1995; 80: 408-14.
[4] Ruth K, Grubelnik A, Hartmann R, Egli T, Zinn M, Ren Q. Efficient production of (R)-3-hydroxycarboxylic acids by biotechnological conversion of polyhydroxyalkanoates and their purification. Biomacromolecules 2007; 8: 279-86.
[5] Merrifield B. Solid phase synthesis. Science. 1986; 232: 341 - 7.
[6] Falchi A, Giacomelli G, Porcheddu A, Taddei M. 4-(4,6-Dimethoxy[1,3,5]triazin-2-yl)-4-methyl-morpholinium chloride (DMTMM): a valuable alternative to PyBOP for solid phase peptide synthesis. Synlett 2000; 2: 275-7.
[7] Kaiser E, Colescott RL, Bossinger CD, Cook PI. Color test for detection of free terminal amino groups in the solid-phase synthesis of peptides. Anal Biochem 1970; 34: 595-8.
[8] Hsu M-Y, Elder DE, Herlyn M. Melanoma: the Wistar (WM) melanoma cell lines. In: Masters JRW, Palsson B, editors. Human Cell Culture. London: Kluwer Academic Publishers; 1999. p. 259-74.
[9] Hansen MB, Nielsen SE, Berg K. Re-examination and further development of a precise and rapid dye method for measuring cell growth/cell kill. J Immunol Methods 1989; 119: 203-10.
[10] Pegram MD, Konecny GK, O'Callaghan C, Beryt M, Pietras R, Slamon DJ. Rational combinations of Trastuzumab with chemotherapeutic drugs used in the treatment of breast cancer. J Nat Cancer Institute 2004; 96: 739-49.

## Claims

**1.** A conjugate of a D-peptide and a (R)-3-substitutedfatty acid, in which the D-peptide has a sequence of SEQUENCE ID NO: 1 or a cytotoxic variant thereof having at least 80% sequence identity with SEQUENCE ID NO: 1.

**2.** A conjugate according to Claim 1 in which the (R)-3-substituent is selected from hydroxyl, nitro, halogen, or mesylate.

**3.** A conjugate according to Claim 2 in which the (R)-3-substituted fatty acid is a (R)-3-hydroxylated fatty acid.

**4.** A conjugate according to any preceding Claim, in which the fatty acid is a C₆ to C₁₂ fatty acid.

**5.** A conjugate according to Claim 4, in which the fatty acid is a C₈ to C₁₂ fatty acid.

**6.** A conjugate as claimed in any preceding Claim in which the peptide has at least 90% sequence identity with SEQUENCE ID NO: 1.

**7.** A conjugate as claimed in Claim 6 in which the peptide is selected from SEQUENCE ID NO: 1, SEQUENCE ID NO: 2, or SEQUENCE ID NO: 3.

**8.** A conjugate as claimed in any preceding Claim in which the peptide is conjugated to the fatty acid by means of an amide linkage between a carboxyl of the medium chain fatty acid and an N-terminal amino of the peptide.

**9.** A conjugate according to Claim 1 and comprising a D-peptide of SEQUENCE ID NO: 1, SEQUENCE ID NO: 2, or SEQUENCE ID NO: 3, conjugated to a (R)-3-substituted C₆ to C₁₂ fatty acid by means of an amide linkage between a carboxyl of the fatty acid and an N-terminal amino of the D-peptide.

**10.** A pharmaceutical composition comprising a conjugate of any of Claims 1 to 9 and a pharmaceutically acceptable carrier.

**11.** A pharmaceutical composition according to Claim 10 further including a chemotherapeutic agent.

**12.** A conjugate according to any of Claims 1 to 9 in the form of a pharmaceutically acceptable salt.

**14.** A conjugate according to any of Claims 1 to 9 for use in a method of treating or preventing cancer in a mammal.

**15.** A conjugate according to any of Claims 1 to 9 and a further chemotherapeutic agent, for use in combination in the treatment or prevention of cancer in a mammal.
